(19) Europäisches Patentamt
European Patent Office
Office européen des brevets

(11) Veröffentlichungsnummer : **0 032 388**
**B1**

(12) # EUROPÄISCHE PATENTSCHRIFT

(45) Veröffentlichungstag der Patentschrift : **05.10.83**

(51) Int. Cl.³ : **C 07 C103/20**, C 07 C103/76, A 61 K 49/04

(21) Anmeldenummer : **81100107.2**

(22) Anmeldetag : **09.01.81**

(54) **Ionische 5-C-substituierte 2,4,6-trijod-isophthalsäure-Derivate, deren Herstellung und diese enthaltende Röntgenkontrastmittel.**

(30) Priorität : **11.01.80 DE 3001293**

(43) Veröffentlichungstag der Anmeldung : **22.07.81 Patentblatt 81/29**

(45) Bekanntmachung des Hinweises auf die Patenterteilung : **05.10.83 Patentblatt 83/40**

(84) Benannte Vertragsstaaten : **AT BE CH DE FR GB IT LI LU NL SE**

(56) Entgegenhaltungen :
**CH A 525 677**
**DE A 2 031 724**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Gries, Heinz, Dr.**
**Helmstädter Strasse 19**
**D-1000 Berlin 31 (DE)**
Erfinder : **Pfeiffer, Heinrich, Dr.**
**Pulfrichzeile 9**
**D-1000 Berlin 20 (DE)**
Erfinder : **Speck, Ulrich, Dr.**
**Benediktiner Strasse 50**
**D-1000 Berlin 28 (DE)**
Erfinder : **Mützel, Wolfgang, Dr.**
**Weddigenweg 74**
**D-1000 Berlin 45 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 032 388 B1

Ionische 5-C-substituierte 2,4,6-trijod-isophthalsäure-Derivate, deren Herstellung und diese enthaltende Röntgenkontrastmittel

Die Erfindung betrifft neue, ionische, in 5-Stellung C-substituierte 2,4,6-Trijod-isophthalsäure-Verbindungen der allgemeinen Formel I

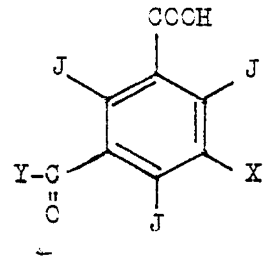

(I)

worin

X den Rest $—CONR_1R_2$, $—CH_2NH$ Acyl oder $—CH_2OH$,

Y den Rest $—NR_3R_4$, NH · Acyl oder $OR_5$ darstellen,

$R_1$, $R_2$, $R_3$ und $R_4$ gleich oder verschieden sind und ein Wasserstoffatom oder einen gegebenenfalls mono- oder polyhydroxylierten gerad- oder verzweigtkettigen niederen Alkylrest,

$R_5$ einen gegebenenfalls mono- oder polyhydroxylierten gerad- oder verzweigtkettigen niederen, Alkylrest und

Acyl den Rest einer gegebenenfalls hydroxylierten niederen aliphatischen Carbonsäure (wobei in den Alkylresten $R_1$, $R_2$, $R_3$, $R_4$, $R_5$ und im Acylrest anwesende OH-Gruppen auch funktionell abgewandelt sein können) bedeuten, und deren Salze, sowie ein Verfahren zu deren Herstellung und neue Röntgenkontrastmittel, die Verbindungen der Formel I als schattengebende Substanz enthalten.

Die unsubstituierten Alkylgruppen $R_1$ bis $R_5$, die gerad- oder verzweigtkettig sein können, enthalten 1-6, vorzugsweise 1-4, insbesondere 1-2 Kohlenstoffatome. Beispielsweise genannt seien insbesondere der Methyl-, Äthyl- und Propylrest ; bevorzugt ist der Methylrest.

Ist der Alkylrest ein Mono- oder Polyhydroxyalkylrest, kann er gerad- oder verzweigtkettig sein. Bevorzugt geeignet sind Alkylreste mit 2-8, vorzugsweise mit 2-4 Kohlenstoffatomen. Die Hydroxylgruppen im Alkylrest können als primäre und/oder sekundäre und/oder tertiäre Hydroxylgruppen vorliegen. Der Alkylrest kann 1-5, vorzugsweise 1-3 Hydroxylgruppen enthalten. Beispielsweise seien der Trishydroxymethylmethylrest, der 1,3,4-Trihydroxy-n-butyl-(2)-rest und bevorzugt der Bishydroxymethylrest, insbesondere der Hydroxyäthyl und der 2,3-Dihydroxypropylrest genannt. Bedeutet der Substituent X die Gruppe $—CH_2NHAcyl$ und/oder Y den Rest —NH · Acyl, so leitet sich der Acylrest von einer aliphatischen Carbonsäure mit 2 bis 6 Kohlenstoffatomen ab. Geeignet sind insbesondere aliphatische Carbonsäurereste mit 2-4 Kohlenstoffatomen wie beispielsweise der Propionylrest und vorzugsweise der Acetylrest.

Bevorzugt geeignet sind Acylreste, die durch 1-5, vorzugsweise 1-3 Hydroxylgruppen substituiert sind. Beispielsweise genannt sei der Hydroxypropionylrest, bevorzugt ist der Hydroxyacetylrest.

Liegen die Hydroxylgruppen im Acylrest und in den Alkylgruppen in funktionell abgewandelter Form vor, so liegen sie vorzugsweise als Äthergruppen, z. B. als Methoxygruppe, vor Beispielsweise genannt sei die Methoxyacetylgruppe und die 2-Methoxyäthylgruppe.

Sollen die Verbindungen der Formel I in Form ihrer physiologisch verträglichen Salze angewendet werden, so kommen zur Salzbildung die dem Fachmann dafür bekannten sowohl anorganischen als auch organischen Basen infrage. Die Herstellung der Salze erfolgt durch Umsetzung der entsprechenden Säure mit der Base in an sich bekannter Weise.

Als physiologisch verträgliche Salze mit Basen kommen somit sowohl Metallsalze, wie zum Beispiel Natrium-, Lithium-, Calcium und Magnesiumsalze, infrage als auch Aminsalze, wie beispielsweise Glucamin-, N-Methylglucamin-, N,N-Dimethylglucamin-, Äthanolamin-, Diäthanolamin-, Morpholinsalze u. a.

Geeignet sind auch Salze basischer Aminosäuren, wie beispielsweise Lysin-, Ornithin-, Arginin- Salze o. ä.

Seit Einführung der trijodierten Benzoesäurederivate als schattengebende Substanzen in Kontrastmittel für die Darstellung von Blutgefäßen, ableitenden Harnwegen und anderen Körperhöhlen und Geweben zur Röntgendiagnostik sind eine Vielzahl von Derivaten synthetisiert, geprüft und zum Teil auch praktisch angewandt worden.

Nahzu alle bisher beschriebenen Verbindungen leiten sich von den beiden Grundstrukturen Trijoddiaminobenzoesäure und Trijodaminoisophthalsäure ab.

Die Derivate beider Grundkörper entsprechen den immer höheren Anforderungen an ein ideales Röntgenkontrastmittel nicht. Die wichtigsten Eigenschaften sind hohe Kontrastdichte, chemische Stabilität und möglichst völlige Untoxizität des Wirkstoffs, niedrige Viskosität der flüssigen Zubereitung und an die Applikationsform angepaßte pharmakodynamische Eigenschaften. Das « ideale Kontrastmittel » sollte alle diese Eigenschaften in sich vereinigen.

Die relativ gute Verträglichkeit der heute gebräuchlichen Röntgenkontrastmittel wird dadurch erreicht, daß die lipophilen und toxischen Grundkörper durch stark hydrophile Substituenten entgiftet werden. Andererseits ist bekannt, daß durch die Anforderungen an ein ideales Kontrastmittel bezüglich Kontrastdichte, Stabilität und Viskosität die Variationsmöglichkeiten der beiden genannten Grundstrukturen erheblich eingeschränkt sind, insbesondere auch im Hinblick darauf, daß für den praktischen Gebrauch nur Substanzen mit hohem Jodgehalt infrage kommen.

Da die Synthesemöglichkeiten inzwischen weitgehend erschöpft sind, ist die Einführung einer neuen Grundstruktur von besonderen Wert.

Der vorliegenden Erfindung lag daher die Aufgabe zugrunde, neue Grundstrukturen für Röntgenkontrastmittel zu synthetisieren, die selbst schon möglichst hydrophil und untoxisch sind. Die vorliegende Erfindung umfaßt ionische Röntgenkontrastmittel auf Basis schattengebender Substanzen mit neuen Grundstrukturen. Diese neuen erfindungsgemäßen schattengebenden Substanzen sind durch eine Reihe von Vorteilen ausgezeichnet :

Sie leiten sich ab von trijodierten Aromaten als Grundkörper, die selbst schon hydrophil und relativ untoxisch sind. Dadurch kann man auf die Einführung relativ schwerer hydrophiler Substituenten zur Verminderung der Chemotoxizität verzichten, wodurch die erfindungsgemäßen Verbindungen einen hohen Jodgehalt besitzen, der bei einigen Vertretern dieser Verbindungsklasse sogar über dem der Diatrizoesäure und der Iothalamsäure liegt.

Im Gegensatz zu den Röntgenkontrastmitteln, die sich von Trijoddiaminobenzoesäure und Trijodamino-isophthalsäure ableiten, besitzen die erfindungsgemäßen Verbindungen keine Aminogruppe, die die neurale Verträglichkeit beeinträchtigt. Die Substitution der aromatischen Aminogruppe durch hydrophile Kohlenstoff-Substituenten hat die Allgemeinverträglichkeit der erfindungsgemäßen Verbindungen wesentlich erhöht.

In Tabelle 1 werden die bekannten Kontrastmittel Iothalamsäure (E) (Deutsche Offenlegungsschrift 1.443.297) und Diatrizoesäure (F) (US-Patentschrift 3.076.024, DBP 970.133, DBP 1.260 477) mit der erfindungsgemäßen 5-N-Methylcarbamoyl-2,4,6-trijodisophthalamsäure (D) im Hinblick sowohl auf die neutrale Verträglichkeit nach intracerebraler bzw. intrazisternaler Verabreichung als auch auf ihre Allgemeinverträglichkeit verglichen.

Die Substanzen werden, als Megluminsalze in Wasser gelöst, je 10 Ratten in unterschiedlicher Dosierung verabreicht. Nach intracerebraler und intrazisternaler Injektion werden die Tiere 24 Stunden beobachtet ; als $ED_{50}$ wird diejenige Dosis bezeichnet, die bei 50 % der Tiere toxische Wirkungen (starke Haltungsanomalien und Störungen der Bewegungskoordination, Krämpfe oder Tod) hervorruft. Nach intravenöser Injektion betrug die Beobachtungszeit 7 Tage. $ED_{50}$ und $LD_{50}$ wurden mittels Probitanalyse berechnet.

Tabelle I

Neurale und allgemeine Verträglichkeit von Derivaten der Trijodtrimesinsäure im Vergleich zur Iothalamsäure (E) und Diatrizoesäure (F)

| Substanz | Jod-Gehalt der Säure [%] | Verabreichung intracerebral, Ratte $Ed_{50}$ mg J/kg | $LD_{50}$ mg J/kg | intrazisternal, Ratte $ED_{50}$ mg J/kg | $LD_{50}$ mg J/kg | intravenös Ratte $LD_{50}$ g J/kg |
|---|---|---|---|---|---|---|
| D | 63,5 | 42 | 66 | 13,2 | 65 | 8,7 |
| E | 62,1 | 36 | 52 | 10,6 | 58 | 7,4 |
| F | 62,1 | 12 | | 3,0 | | 7,3 |

Wegen der guten Verträglichkeit sind die erfindungsgemäßen Verbindungen für alle Anwendungen geeignet, in denen jodhaltige, nierengängige Kontrastmittel eingesetzt werden. Wegen der guten Allgemeinverträglichkeit ist insbesondere die Anwendung nach i. v. Injektion in der Urographie und in der Computer-Tomographie vorgesehen.

In der Urographie ist zusätzlich die erwünschte osmodiuretische Wirkung zur Füllung der ableitenden Harnwege von Vorteil.

Die Erfindung betrifft somit auch neue Röntgenkontrastmittel auf Basis von Verbindungen der allgemeinen Formel I.

Die Herstellung der neuen Röntgenkontrastmittel auf Basis der erfindungsgemäßen Verbindungen der allgemeinen Formel (I) erfolgt in an sich bekannter Weise, z. B. dadurch, daß man die schattengebende Substanz mit den in der Galenik üblichen Zusätzen, z. B. Stabilisatoren wie Natriumedetat, Calcium-di-natriumedetat, physiologisch verträglichen Puffern, Natriumchlorid u. ä., in eine für die intravenöse Applikation geeignete Form bringt. Die Konzentration der neuen Röntgenkontrastmittel im

wäßrigen Medium richtet sich nach der röntgendiagnostischen Methode. Die bevorzugten Konzentrationen und Dosierungen der neuen Verbindungen bewegen sich in den Bereichen von 50-400 mg J/ml für die Konzentration und 5-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, dadurch gekennzeichnet, daß man in an sich bekannter Weise

a) eine Verbindung der allgemeinen Formel A

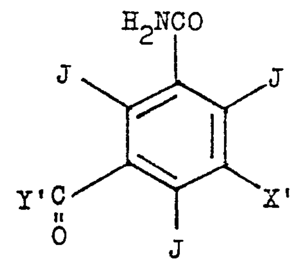

(A)

worin

X' die Gruppen $—CONR_1'$, $R_2'$, $—CH_2OH$ oder $—CH_2NHAcyl$,

Y' die Gruppen $—NR_3'R_4'$, —NHAcyl, oder $—OR_5$ bedeuten und

$R_1'$, $R_2'$, $R_3'$, $R_4'$, $R_5$ und Acyl die oben angegebenen Bedeutungen für $R_1$, $R_2$, $R_3$, $R_4$ und $R_5$ besitzen, jedoch sowohl $R_1'$ und $R_2'$ als auch $R_3'$ und $R_4'$ nicht gleichzeitig Wasserstoff darstellen und Acyl die obengenannte Bedeutung besitzt, mit einem Diazotierungsreagenz umsetzt, oder

b) in einer Verbindung der allgemeinen Formel B

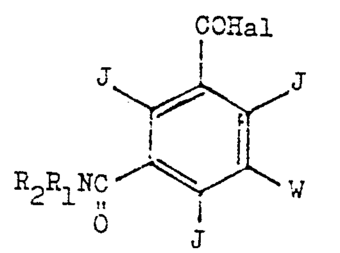

(B)

worin

W die Gruppen $—CONR_3R_4$, —CONHAcyl, $—CH_2OH$ oder $—CH_2NHAcyl$,

Hal ein Brom- oder Chloratom bedeuten und

$R_1$, $R_2$, $R_3$, $R_4$ und Acyl die obengenannten Bedeutungen besitzen, die Carbonsäurehalogenidgruppe hydrolysiert, oder

c) eine Verbindung der allgemeinen Formel C

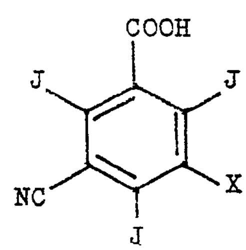

(C)

worin

X die obengenannte Bedeutung besitzt, mit einem reaktionsfähigen Derivat einer aliphatischen Carbonsäure AcylOH umsetzt, worin Acyl die obengenannte Bedeutung besitzt oder der partiellen Hydrolyse unterwirft und gegebenenfalls die so erhaltene 5-Carbamoylverbindung der Formel I, worin $R_1$ und $R_2$ nicht gleichzeitig Wasserstoff sind, diazotiert, anschließend mit einer Base der Formel $HNR_3R_4$ (mit $R_3$ und $R_4$ in der obengenannten Bedeutung) partiell amidiert und das so erhaltene Monocarbonsäurechlorid hydrolysiert und gewünschtenfalls anschließend die erhaltenen Verbindungen der Formel I N-alkyliert und/oder Schutzgruppen abspaltet und/oder durch Umsetzung mit anorganischen oder organischen Basen Salze herstellt.

Die Überführung einer primären Amidgruppe in die Carbonsäure gemäß Verfahrensvariante a) erfolgt durch Methoden, die dem Fachmann bekannt sind.

Beispielsweise hydrolysiert man Carbonsäureamide durch Erwärmen in wäßrigen Mineralsäuren oder in wäßrigen Alkalihydroxidlösungen. Die im erfindungsgemäßen Verfahren bevorzugte Methode ist jedoch die « Diazotierung » der primären Amidgruppe mit Diazotierungsreagenzien wie beispielsweise salpetriger Säure in wäßrigem Milieu, bzw. mit Nitrosylsulfat oder Nitrosylchlorid in wasserfreiem Medium, wobei unter Stickstoffabspaltung die Carbonsäure gewonnen wird. Zur praktischen Durchführung wird die Suspension des Ausgangsmaterials in verdünnter Mineralsäure, beispielsweise Schwefelsäure, vorzugsweise Salzsäure, mit der Lösung des Diazotierungsreagenzes wie beispielsweise der wäßrigen Lösung von Natriumnitrit versetzt oder es wird die Lösung oder Suspension des Ausgangsmaterials in Eisessig, konz. Schwefelsäure, Dimethylformamid o. ä. mit der aus Natriumnitrit und konzentrierter Schwefelsäure hergestellten Nitrosylsulfatlösung oder mit Nitrosylchlorid bei 0 °C bis 5 °C versetzt und nach Beendigung der Diazotierung mit Wasser versetzt. Anschließend wird zweckmäßigerweise die Reaktionslösung einige Zeit zur Stickstoffabspaltung erwärmt.

Zur Herstellung von Verbindungen der allgemeinen Formel I, die in 5-Stellung eine Estergruppe —$COOR_5$ enthalten, geht man zweckmäßigerweise von der freien Carbonsäure aus, die man nach dem Fachmann bekannten Methoden verestert. Enthält der Alkylrest $R_5$ eine oder mehrere Hydroxylgruppen, so setzt man $R_5$ zweckmäßigerweise als Halogenid $R_5Hal$ ein, indem man dieses in einem geeigneten Lösungsmittel, vorzugsweise Dimethylformamid oder Dimethylacetamid, mit dem Alkalisalz der Säure, vorzugsweise dem Natriumsalz, zur Umsetzung bringt.

Neben dieser im Rahmen der vorliegenden Erfindung bevorzugten Veresterungsmethode kann die Veresterung der Carboxylgruppe auch nach anderen dafür gebräuchlichen Methoden vorgenommen werden, besonders dann, wenn der einzuführende $R_5$-Rest keine zusätzlichen Hydroxylgruppen enthält. Beispielsweise genannt sei hier die Veresterung mit Dialkylsulfat oder Diazoalkanen u. ä. oder die Umsetzung der Carboxylgruppe mit einem Alkohol $R_5OH$, vorzugsweise in Gegenwart z. B. einer Mineralsäure wie Schwefelsäure. Die Veresterung der Carboxylgruppe durch die oben beschriebenen Methoden kann in Gegenwart anderer kernständiger Gruppen wie Cyano-, Carbamoyl-, Hydroxy-, methyl-, Amidoacyl-, Methylaminoacyl- außer einer weiteren Carboxylgruppe vorgenommen werden.

Wird im Verlaufe des erfindungsgemäßen Verfahrens (Verfahrensvariante b) die Carbonsäurehalogenidgruppe zur freien Carbonsäure hydrolysiert, so erfolgt diese Reaktion ebenfalls nach bekannten Methoden. Beispielsweise kann man die Carbonsäurehalogenide in wäßrigem Medium, zweckmäßigerweise in Gegenwart eines Lösungsvermittlers wie Dioxan, Aceton, Tetrahydrofuran, vorzugsweise Dimethylsulfoxid und in Gegenwart von tertiären Basen wie Triäthylamin, Pyridin oder von Alkalihydroxid bei einer Temperatur von ca. Raumtemperatur bis 100 °C zu den Carbonsäuren hydrolysieren.

Die Überführung der Cyano-Gruppe (Verfahrensvariante c) in die N-acylierte Amid-Gruppe —CONHAcyl (Acyl hat die oben angegebene Bedeutung) erfolgt durch Anlagerung einer aliphatischen Carbonsäure AcylOH an die CN-Dreifachbindung nach dafür dem Fachmann bekannten Arbeitsmethoden. Zur praktischen Durchführung wird die Säure AcylOH zweckmäßigerweise in Form eines reaktiven Derivates, vorzugsweise als Anhydrid, zur Einwirkung gebracht. Die Umsetzung erfolgt in Gegenwart eines geeigneten sauren Katalysators, wie z. B. Perchlorsäure oder Schwefelsäure, Phosphorsäure und ähnliche. In der Regel dient das eingesetzte Säureanhydrid auch gleichzeitig als Lösungsmittel, was nicht ausschließt, daß man dem Reaktionsgemisch einen geeigneten Lösungsvermittler wie beispielsweise Dioxan zusetzt. Die Umsetzung erfolgt bei Raumtemperatur oder erhöhter Temperatur. Erfolgt die Umsetzung bei erhöhter Temperatur, ist der bevorzugte Tempteraturbereich 40-110 °C.

Die im Verlaufe des beanspruchten Verfahrens notwendige partielle Hydrolyse der Nitrilgruppe zum primären Amid erfolgt nach dem Fachmann bekannten Verfahren. So kann man zum Beispiel die Nitrilgruppe dadurch in das Amid überführen, daß man die Ausgangsverbindung in konzentrierten Mineralsäuren wie Chlorwasserstoffsäure, Schwefelsäure oder Phosphorsäure löst und die Hydrolyse bei Temperaturen von ca. Raumtemperatur bis 100 °C durchführt. Man kann die Nitrilgruppe aber auch in alkalischem Milieu partiell hydrolysieren, indem man die Ausgangsverbindung in wäßrigem Alkalihydroxid löst oder suspendiert und bei einer Temperatur von ca. Raumtemperatur bis 100 °C, vorzugsweise bei 40 °C bis 80 °C hydrolysiert.

Die gewünschtenfalls anschließende Diazotierung, gemäß Verfahrensvariante c, erfolgt in an sich bekannter Weise, wie bei der Verfahrensvariante a beschrieben.

Die partielle Amidierung wird nach bekannten Methoden durchgeführt, indem man die Dicarbonsäure, gewünschtenfalls unter intermediärem Schutz eventuell vohandener freier Hydroxylgruppen gegebenenfalls in einem geeigneten Lösungsmittel wie beispielsweise Benzol, Toluol oder Acetonitril mit einem Chlorierungsreagenz wie Phosphorpentachlorid, Oxalylchlorid, Phosgen oder 1,1-Dichlormethylmethyläther, vorzugsweise Thionylchlorid, bei einer Temperatur von Raumtemperatur bis 100 °C, vorzugsweise von 50 bis 80 °C zum Dicarbonsäurechlorid umsetzt, nach Beendigung der Chlorwasserstoff-Entwicklung im Vakuum einengt und das Dicarbonsäuredichlorid in üblicher Weise isoliert. Die partielle Umsetzung des Dichlorides mit der Base $HN(R_3R_4)$ erfolgt ebenfalls nach bekannten Methoden, indem man z. B. das Dicarbonsäuredichlorid gelöst oder suspendiert in einem geeigneten Lösungsmittel wie beispielsweise Toluol, Dioxan, vorzugsweise Dimethylacetamid in Gegenwart von einem Mol einer tertiären Base wie zum Beispiel Tributylamin, Triäthylamin oder Pyridin, mit der Lösung von einem Mol Base $HNR_3R_4$ (10 % Überschuß) in demselben Lösungsmittel versetzt, bei einer Temperatur von 10 °C bis 100 °C, vorzugsweise von 50 bis 90 °C reagieren läßt und anschließend das Monoamid-monochlorid in üblicher Weise isoliert.

Die nachträgliche N-Alkylierung führt man, nach intermediärem Schutz der Hydroxylgruppen, z. B. in der Weise durch, daß man auf das entsprechende Säureamid zunächst einen Protonenakzeptor wie Natriumamid, Natriumhydrid oder auch Alkalihydroxid einwirken läßt und dann umsetzt mit einem $R_5$-Alkylhalogenid, vorzugsweise als Bromid, oder insbesondere mit einem Dialkylsulfat (z. B. Dimethyl- oder

Diäthylsulfat). Je nach dem verwendeten Protonenakzeptor erfolgt die Umsetzung in wasserfreiem oder wäßrigem Reaktionsmilieu bei einer Reaktionstemperatur von ca. Raumtemperatur bis 100 °C, vorzugsweise 50 bis 70 °C.

Geeignete Lösungsmittel bzw. Lösungsvermittler sind bekanntermaßen Aceton, Dimethylformamid, Dioxan, Tetrahydrofuran u. ä.

Der intermediäre Schutz von freien Hydroxylgruppen erfolgt nach üblichen Methoden durch leicht wieder abspaltbare Schutzgruppen. Die Einführung solcher Gruppen kann durch Acylierung (z. B. Einführung eines vorzugsweisen Acetylrestes oder Benzoylrestes), durch Verätherung (z. B. Einführung des Triphenylmethylrestes) oder durch Acetalisierung oder Ketalisierung z. B. mittels Acetaldehyd, Dihydropyran, Aceton oder 2,2-Dimethoxypropan erreicht werden.

Die spätere Abspaltung der intermediär eingeführten Schutzgruppen, unter Freisetzung der letztlich gewünschten Hydroxylgruppen, erfolgt ebenfalls nach Methoden, die dem Fachmann bekannt sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführtwerden. Acetal-, Ketal- oder Ätherschutzgruppen können beispielsweise durch saure Hydrolyse abgespalten werden.

Die Umsetzung der erfindungsgemäßen Säuren der Formel I zu den physiologisch verträglichen Salzen mit den dem Fachmann dafür gebräuchlichen anorganischen oder organischen Basen erfolgt ebenfalls nach bekannten Methoden.

Die verfahrensgemäß eingesetzten Ausgangsprodukte können nach an sich bekannten Methoden, beispielsweise aus den bekannten Verbindungen der allgemeinen Formel

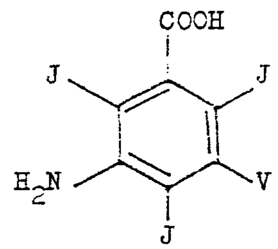

worin

V die Reste $—NH_2$, —COOH, $—CH_2NHAcyl$ oder $—CH_2OH$ bedeuten, hergestellt werden.

Dabei wird in einer Sandmeyer-Reaktion die aromatische Aminogruppe durch die Cyanogruppe substituiert, wie am Beispiel der Herstellung von 3,5-Dicyano-2,4,6-trijod-benzoesäure und 5-Cyano-2,4,6-trijod-isophthalsäure näher erläutert werden soll :

3,5-Dicyano-2,4,6-trijod-benzosäure

7 g Natriumnitrit werden unter Rühren in 84 ml einer auf + 5 °C temperierten konzentrierten Schwefelsäure eingetragen. Man hält anschließend solange bei + 70 °C, bis Lösung eingetreten ist und kühlt dann auf + 5 °C ab. Nach Zutropfen von 42 ml Eisessig unter Kühlen trägt man portionsweise 21 g 3,5-Diamino-2,4,6-trijod-benzoesäure unter Rühren so ein, daß die Innentemperatur zwischen 0 °C und + 5 °C liegt. Man läßt den Ansatz noch 2 Stunden nachrühren und gießt die grüngefärbte Suspension auf 400 g Eis auf. Man versetzt 500 ml konz. Ammoniak mit 320 ml Wasser und löst darin 35,6 g Kupfer-I-cyanid und 67 g Kaliumcyanid. Zu dieser Lösung gibt man den Diazotierungsansatz, wobei starkes Schaumen eintritt. Man läßt noch 2 Stunden nachrühren und versetzt den Ansatz nach Stehen über Nacht zuerst mit 500 ml Essigester, dann mit überschüssiger Konzentrierter Salzsäure. Nach Absaugen der ausgeschiedenen Kupfersalze, die mit Essigester ausgewaschen werden, trennt man im Filtrat die wäßrige Phase ab und extrahiert sie mehrfach mit Essigester nach. Die Essigesterextrakte werden vereinigt, mit Wasser gewaschen und anschließend über Natriumsulfat getrocknet und eingeengt. Der dunkelgefärbte Rückstand wird heiß mit 100 ml Aceton behandelt, die acetonische Lösung von ungelösten Anteilen filtriert und anschließend auf die Hälfte eingeengt. Nach mehrstündigem Rühren wird das Kristallisat abgesaugt, mit eiskaltem Aceton gewaschen und bei 50 °C getrocknet. Man erhält 8,5 g (= 38 % der Theorie) 3,5-Dicyano-2,4,6-trijod-benzoesäure als weißes Pulver mit einem über 280 °C liegenden Zersetzungspunkt.

5-Cyano-2,4,6-trijod-isophthalsäure

112 g 5-Amino-2,4,6-trijod-isopthalsäure werden in 1 100 ml Wasser suspendiert und durch Zugabe von 10 g Ätznatron in Lösung gebracht. Dann kühlt man die Lösung, die durch Zugabe von verd. Schwefelsäure auf einen pH von 2,5 eingestellt wurde, auf 0 °C ab und tropft unter Kühlung eine Lösung von 20 g Natriumnitrit in 60 ml Wasser zu, wobei die Reaktionstemperatur bei 0 bis 5 °C gehalten wurde. Dann stellt man den pH-Wert durch Zutropfen von verdünnter Schwefelsäure erneut auf 2,5 ein und rührt

unter Eiskühlung eine bis 2 Stunden nach. Durch langsames Zutropfen von verdünnter Natronlauge wird der entstandene Niederschlag bei pH 4,5 in Lösung gebracht.

Inzwischen hat man eine 30 °C warme Lösung von 99 g Kupfer-I-chlorid und 172 g Kaliumcyanid in 800 ml Wasser bereitet und setzt die neutralisierte Diazoniumsalzlösung in einem Zug zu, wobei starkes Aufschäumen eintritt. Man läßt noch 15 Minuten bei 30 °C nachrühren und trennt dann die Kupfersalze durch Ansäuern der Reaktionslösung auf pH 3 mit verdünnter Schwefelsäure ab. Das Filtrat wird durch Zugabe weiterer verdünnter Schwefelsäure auf pH 0,5 bis 1 gebracht und der Niederschlag nach mehrstündigem Rühren im Eisbad abgesaugt, mit Wasser gewaschen und bei 50 °C getrocknet. Zur Reinigung suspendiert man das Rohprodukt in 400 ml Wasser, löst durch Zugabe von Natronlauge, behandelt die Lösung mit 10 g Aktivkohle, läßt 30 Minuten bei Raumtemperatur rühren und versetzt das Filtrat mit einem Überschuß an Mineralsäure. Nach mehrstündigem Rühren im Eisbad saugt man den Niederschlag ab, wäscht ihn mit Wasser und trocknet ihn bei 50 °C. Man erhält 89 g (78 % der Theorie) 5-Cyano-2,4,6-trijod-isophthalsäure als weißes Pulver mit einem Zersetzungspunkt oberhalb 300 °C.

In analoger Weise werden aus den entsprechenden Amino-Verbindungen hergestellt :

5-Cyano-3-N,N-dimethylcarbamoyl-2,4,6-trijod-benzoesäure ; Fp. : 240 °C (Zersetzung) ; Ausbeute : 85 % der Theorie.

5-Cyano-3-(N-methylcarbamoyl)-2,4,6-trijod-benzoesäure ; Fp. : 300 °C (Zersetzung) ; Ausbeute : 72 % der Theorie.

5-Cyano-3-[N-methyl-N-(2-hydroxyäthyl)-carbamoyl]-2,4,6-trijod-benzoesäure ; Fp. : 280 °C (Zersetzung) ; Ausbeute : 89 % der Theorie.

5-Cyano-3-carbamoyl-2,4,6-trijod-benzoesäure ; Fp. : > 300° (Zersetzung) ; Ausbeute : 82 % der Theorie.

5-Cyano-3-[N-(2-hydroxyäthyl)-carbamoyl]-2,4,6-trijod-benzoesäure ; Fp. : > 300 °C (Zersetzung) ; Ausbeute : 95 % der Theorie.

5-Cyano-3-acetylaminomethyl-2,4,6-trijod-benzoesäure. Fp. : 271 °C (Zersetzung) ; Ausbeute : 85 % der Theorie.

5-Cyano-3-hydroxymethyl-2,4,6-trijod-benzoesäure ; Fp. : 250-252 °C (Zersetzung) ; Ausbeute : 81 % der Theorie.

Wie bereits oben ausgeführt, erfolgt die Veresterung der Carboxylgruppe nach an sich bekannten Methoden, wobei Methoden, wie sie für die Veresterung von trijodierten Aminobenzoesäure-Derivaten beschrieben sind, bevorzugt werden. Die Veresterung wird in den nachfolgenden Herstellungsvorschriften erläutert :

5-Cyano-3-N-methylcarbamoyl-2,4,6-trijod-benzoesäure-(2,3-dihydroxy-propyl)-ester

60 g 5-Cyano-3-N-methylcarbamoyl-2,4,6-trijod-benzoesäure werden in 150 ml Dimethylformamid mit 22 g Natriumcarbonat (wasserfrei) und 28 g 1-Chlorpropandiol-(2,3) 4 Stunden auf 90 °C erwärmt. Danach kühlt man ab, saugt vom ausgeschiedenen Kochsalz ab und engt im Vakuum zur Trockne ein. Der Rückstand wird in 250 ml Essigester gelöst, die Lösung über Aktivkohle filtriert und das Filtrat auf die Hälfte eingeengt. Man kühlt im Eisbad ab und versetzt vorsichtig mit soviel Diisopropyläther, daß Kristallisation einsetzt. Nach mehrständigem Nachrühren im Eisbad wird das Kristallisat abgesaugt, mit Diisopropyläther gewaschen und bei 50 °C getrocknet. Man erhält 48,5 g (= 74 % der Theorie) 5-Cyano-3-N-methylcarbamoyl-2,4,6-trijodbenzoesäure-(2,3-dihydroxypropyl)-ester als weißes Pulver vom Fp. : 117-120 °C.

5-Carbamoyl-3-N-methylcarbamoyl-2,4,6-trijod-benzoesäure-(2,3-dihydroxypropyl)-ester

24 g 5-Carbamoyl-3-N-methylcarbamoyl-2,4,6-trijod-benzoesäure werden in 200 ml Wasser suspendiert und durch Zugabe von konzentrierter Natronlauge bei pH 7 in Lösung gebracht. Man engt im Vakuum zur Trockne ein und löst das Natriumsalz in 60 ml Dimethylformamid. Dann gibt man 10 g 1-Chlorpropandiol (2,3) zu und läßt 6 Stunden bei 90 °C rühren. Nach Abkühlen auf Raumtemperatur wird das ausgeschiedene Natriumchlorid abgesaugt und das Filtrat im Vakuum zur Trockne gebracht. Der Rückstand wird mit 40 ml Wasser 2 Stunden ausgerührt. Der Niederschlag wird abgesaugt, mit Wasser gewaschen und bei 50 °C getrocknet. Man erhält 23,5 g (87 % der Theorie) 5-Carbamoyl-3-N-methylcarbamoyl-2,4,6-trijod-benzoesäure-(2,3-dihydroxypropyl)-ester, Fp. : 258 °C (Zersetzung).

Geht man bei der Herstellung der Ausgangsprodukte von Cyano-Vorprodukten aus (Verfahrensvariante c), verseift man in an sich bekannter Weise und wie oben schon beschrieben, die Cyanogruppe in saurem oder alkalischem Milieu zum primären Amid. Diese Reaktion sei am Beispiel der Verseifung von 5-Cyano-2,4,6-trijod-isophthalsäure näher erläutert :

5-Carbamoyl-2,4,6-trijod-isophthalsäure

100 g 5-Cyano-2,4,6-trijod-isophthalsäure werden in 400 ml Wasser suspendiert und durch Zugabe

von 20 g Natriumhydroxid in Lösung gebracht. Die Lösung wird 3 Stunden lang auf + 60 °C gehalten, anschließend unter Rühren in 60 ml konzentrierte Salzsäure eingegossen. Nach mehrstündigem Rühren im Eisbad wird der ausgeschiedene Niederschlag abgesaugt, mit wenig eiskaltem Wasser gewaschen und bei 50 °C getrocknet. Man erhält 98 g 5-Carbamoyl-2,4,6-trijod-isophthalsäure als weißes Pulver mit einem Zersetzungspunkt oberhalb 280 °C.

Dieselbe Verbindung wird auch durch saure Verseifung der Nitrilgruppe in folgender Weise erhalten ;

100 g 5-Cyano-2,4,6-trijod-isophthalsäure werden in 400 ml konzentrierter Schwefelsäure suspendiert und zunächst unter Rühren 30 Minuten auf 60 °C, darauf 2 Stunden auf 95 °C erwärmt. Dann wird die entstandene klare Lösung auf 1,2 kg Eis aufgetragen und eine Stunde im Eisbad nachgerührt. Das ausgeschiedene Rohprodukt wird abgesaugt, in 400 ml Äthanol heiß gelöst und die Lösung mit 70 ml konzentrierter Natronlauge versetzt. Nach mehrstündigem Rühren im Eisbad wird das ausgeschiedene Natriumsalz abgesaugt, mit Äthanol gewaschen und anschließend in 200 ml heißem Wasser gelöst. Nach Behandeln der Lösung mit Aktivkohle wird das Filtrat in 150 ml halbkonzentrierter Schwefelsäure eingefällt. Nach mehrstündigem Rühren im Eisbad wird der Niederschlag abgesaugt, mit wenig eiskaltem.

Wasser gewaschen und bei 50 °C getrocknet. Man erhält 84 g (= 82 % der Theorie) 5-Carbamoyl-2,4,6-trijodisophthalsäure, in ihren Eigenschaften mit dem Produkt der alkalischen Verseifung übereinstimmend.

In analoger Weise werden die folgenden Verbindungen aus den entsprechenden Cyano-Vorprodukten hergestellt :

5-Carbamoyl-2,4,6-trijod-isophthalsäure-bis-(N-methylamid)., Fp : > 300 °C ; Ausbeute : 82 % der Theorie ;

5-Carbamoyl-2,4,6-trijod-isophthalsäure-bis-[N-(2,3-dihydroxypropyl)-amid], Fp. : > 300 °C (Zersetzung) ; Ausbeute : 70 % der Theorie ;

3-[N-(2-Hydroxyäthyl)-carbamoyl]-5-N-methyl-carbamoyl-2,4,6-trijod-benzoesäureamid, Fp. : 300 °C ; Ausbeute : 75 % der Theorie ;

3,5-Bis-[N-(2-hydroxyäthyl)-carbamoyl]-2,4,6-trijodbenzoesäureamid, Fp. : 300 (Zersetzung) ; Ausbeute : 75 % der Theorie ;

3-[N-(2,3-Dihydroxypropyl)-carbamoyl]-5-[N-methyl-N-(2,3-dihydroxypropyl)-carbamoyl]-2,4,6-trijod-benzoesäureamid, Fp. : 202 °C ; Ausbeute : 61 % der Theorie ;

5-Carbamoyl-2,4,6-trijod-isophthalsäure-bis-[N-(2-hydroxyäthyl)-amid], Fp. : 300 °C ; Ausbeute : 75 % der Theorie ;

5-Carbamoyl-2,4,6-trijod-isophthalsäure-bis-[N-methyl-N-(2,3-dihydroxypropyl)-amid] ; Fp. : > 193 °C ; Ausbeute : 50 % der Theorie ;

5-Carbamoyl-2,4,6-trijod-isophthalsäure-bis-[N,N-(2-hydroxyäthyl)-amid] ; Fp. : 198-200 °C, Ausbeute : 69 % der Theorie ;

5-Carbamoyl-2,4,6-trijod-isophthalsäure-bis-[N-(trishydroxymethyl)-methyl-amid], Fp. : > 280 °C ; Ausbeute : 67 % der Theorie.

Die Herstellung der Dicarbonsäuredichloride, die für die Amidierungsreaktion erforderlich sind und der Monocarbonsäurechloride, die als Ausgangsprodukte benötigt werden, aus den entsprechenden Carbonsäure-Vorprodukten, erfolgt nach dem Fachmann bekannten Methoden, die oben schon beschrieben sind und anhand der folgenden Herstellungsvorschrift erläutert werden :

5-Carbamoyl-2,4,6-trijod-isophthalsäure-dichlorid

59,9 g 5-Carbamoyl-2,4,6-trijod-isophthalsäure werden mit 500 ml Thionylchlorid und 0,5 ml Dimethylformamid 4 Stunden unter Rückfluß gerührt. Das Reaktionsgemisch wird danach im Vakuum eingeengt und der Rückstand eine Stunde mit 300 ml Methylenchlorid gerührt. Danach wird das Rohprodukt abfiltriert und bei 50 °C im Vakuum getrocknet. Man rührt dann noch eine Stunde mit 85 ml Aceton, saugt ab und trocknet bei 50 °C im Vakuum. Man erhält 36,6 g 5-Carbamoyl-2,4,6-trijod-isophthalsäuredichlorid mit einem Zersetzungspunkt von 247-248 °C ; Ausbeute : 58 % der Theorie.

In analoger Weise werden aus den entsprechenden Carbonsäurevorstufen hergestellt :

5-N-Methylcarbamoyl-2,4,6-trijod-isophthalsäure-dichlorid ; Fp. : 214-216 °C ; Ausbeute : 48 % der Theorie.

5-N,N-Dimethylcarbamoyl-2,4,6-trijod-isophthalsäure-dichlorid ; Fp. : 272-273 °C ; Ausbeute : 85 % der Theorie.

5-Cyano-2,4,6-trijod-isophthalsäure-dichlorid ; Fp. : 246-264 °C ; Ausbeute : 94 % der Theorie.

5-[N-(2-Acetoxyäthyl)-carbamoyl]-2,4,6-trijod-isophthalsäure-dichlorid ; Fp. : 75-85 °C, Ausbeute : 93 % der Theorie.

5-Cyano-3-N-methyl-carbamoyl-2,4,6-trijod-benzoylchlorid

58,2 g 5-Cyano-3-N-methyl-carbamoyl-2,4,6-trijod-benzoesäure werden in 400 ml Thionylchlorid unter Zusatz von 0,3 ml Dimethylformamid unter Rühren eine Stunde zum Sieden erhitzt. Anschließend wird filtriert und im Vakuum zur Trockne eingedampft. Der Rückstand bildet einen festen Schaum. Zur Reinigung wird in 625 ml Dioxan durch Erwärmen auf dem Dampfbad gelöst, mit 6,3 g Kohle 10 Minuten auf dem Dampfbad behandelt, filtriert, auf ca. 1/5 eingeengt und gekühlt. Der Niederschlag wird abgesaugt und im Vakuum bei 50 °C über KOH-Plätzchen getrocknet. Ausbeute : 33,8 g (56,3 % der Theorie) 5-Cyano-3-N-methyl-carbamoyl-2,4,6-trijod-benzoylchlorid ; Fp. 265°-267 °C.

Die Amidierung der beiden 1- und 3-ständigen Säurechloridgruppen kann in einem Reaktionsschritt erfolgen, wobei Bisamide entstehen, oder auch stufenweise durchgeführt werden, nach Verfahren die dem Fachmann bekannt sind. Sind die beiden 1- und 3-ständigen Amidreste im letztlich gewünschten Verfahrensprodukt bezüglich der N-Substituenten gleich ($R_1 = R_3$ und $R_2 = R_4$), erfolgt die Amidierung vorzugsweise in einem Reaktionsschritt. Unterscheiden sich jedoch diese beiden Amidgruppen bezüglich der N-Substituenten $R_1$ bis $R_4$, so erfolgt die Amidierung vorzugsweise stufenweise. Die bei der stufenweisen Amidierung erhaltenen Monoamide-monochloride sind Ausgangsverbindungen für die Verfahrensvariante b) ; sie können aber auch mit der Base $HNR_3R_4$ umgesetzt werden zu gewünschten Bisamiden mit $—CONR_1R_2 \neq —CONR_3R_4$.

Dabei isoliert man zweckmäßigerweise das in erster Stufe nach Umsetzung des Dicarbonsäuredichlorides mit der organischen primären oder sekundären Base $HN(R_1R_2)$ erhaltene Monoamid-Monochlorid in üblicher Weise, um unerwünschte Nebenreaktionen zu vermeiden und setzt es danach, falls erwünscht, mit der organischen primären oder sekundären Base $HN(R_3R_4)$ um. Zur Herstellung von Ausgangsprodukten, in denen die einzuführenden Amidgruppen gleich sind, wird das gelöste Dicarbonsäuredichlorid umgesetzt mit 4 Äquivalenten der organischen sekundären oder primären Base $HN(R_1R_2)$ oder mit 2 Äquivalenten dieser Base und in Gegenwart von 2 Äquivalenten einer tertiären Base, wie beispielsweise Pyridin, Triäthylamin, vorzugsweise Tributylamin.

Die nachfolgenden Beispiele sollen die Amidierung in einem Reaktionsschritt erläutern :

5-Carbamoyl-2,4,6-trijod-isophthalsäure-bis-[1,1-bis-(hydroxymethyl)-methylamid]

11 g 5-Carbamoyl-2,4,6-trijod-isophthalsäure-dichlorid werden in 22 ml Dimethylacetamid (DMA) gelöst und auf + 50 °C erwärmt und unter Rühren innerhalb 5 Minuten mit einer Lösung von 40 g Serinol (2-Amino-1,3-dihydroxypropan) in 15 ml DMA versetzt, wobei die Temperatur auf + 62 °C steigt. Anschließend werden 10,5 ml Tributylamin zugegeben, 4 Stunden bei ca. 50 °C und über Nacht bei Raumtemperatur gerührt. Es werden 2,4 ml konzentrierte Salzsäure bis zur sauren Reaktion zugetropft und die Lösung wird dann tropfenweise in 270 ml Methylenchlorid eingerührt. Nach einstündigem Rühren wird vom klebrigen Niederschlag dekantiert und nochmals 30 Minuten mit 135 ml Methylenchlorid ausgerührt. Das Rohprodukt (14,9 g) wird in 110 ml Wasser gelöst und über eine Säule mit ca. 200 ml Kationenaustauscher IR 120 gegeben. Aus dem wäßrigen Eluat wird 12,5 g erhalten. Sie werden in 120 ml Wasser gelöst und über eine Säule mit ca. 200 ml Anionenaustauscher IRA 410 gegeben. Das Eluat wird 30 Minuten mit 1,2 g Kohle gerührt, filtriert, im Vakuum eingeengt und bei 50 °C im Vakuum getrocknet. Ausbeute : 8,4 g 5-Carbamoyl-2,4,6-trijod-isophthalsäure-bis-[1,1-bis(hydroxymethyl)-methylamid] ; Fp. : 246-252 °C ; Ausbeute : 65 % der Theorie.

5-Cyano-2,4,6-trijod-isophthalsäure-bis-[(2-hydroxyäthyl)-amid]

151,4 g 5-Cyano-2,4,6-trijod-isophthalsäure-dichlorid werden als Suspension in 1,5 l Dioxan innerhalb von 25 Minuten mit einer Lösung von 75,5 ml Äthanolamin in 750 ml Dioxan versetzt (Wärmetönung bis + 45 °C). Nach Rühren über Nacht wird mit 1,5 l Wasser verrührt und das Dioxan im Vakuum abdestilliert. Nach 2-4 stündigem Rühren wird der Niederschlag abgesaugt, mit Wasser gewaschen und bei 50 °C im Vakuum getrocknet. Ausbeute : 151,4 g (= 92,5 % der Theorie) 5-Cyano-2,4,6-trijod-isophthalsäure-bis-[(2-hydroxyäthyl)-amid] ; Fp. : 300 °C.

In analoger Weise werden hergestellt :

5-Cyano-2,4,6-trijod-isophthalsäure-bis-[(2,3-dihydroxypropyl)]-amid ; Fp. : > 280 °C (Zersetzung) ; Ausbeute : 80 % der Theorie ;

5-Cyano-2,4,6-trijod-isophthalsäure-bis-[bis(2-hydroxyäthyl)]-amid, Fp. : 212-215 °C ; Ausbeute : 78 % der Theorie ;

5-Cyano-2,4,6-trijod-isophthalsäure-bis-[tris-(hydroxymethyl)-methyl]-amid, Fp. : > 280 °C ; Ausbeute : 70 % der Theorie.

Die nachfolgenden Beispiele sollen die stufenweise Amidierung erläutern :

5-Cyano-3-[N-(2,3-dihydroxypropyl)-carbamoyl]-2,4,6-trijod-benzoylchlorid

18,1 g 5-Cyano-2,4,6-trijod-isophthalsäure-dichlorid werden in 750 ml Dioxan bei 80 °C mit 7,1 g 1-Aminopropandiol (2,3) versetzt und 2 Stunden gerührt. Dann wird vom ausgeschiedenen Hydrochlorid

des 1-Aminopropandiol (2,3) abgetrennt und das Filtrat im Vakuum eingeengt. Der Rückstand wird mit 800 ml Essigester ausgekocht. Aus dem Extrakt kristallisieren nach dem Einengen und Abkühlen 11,7 g (59 % der Theorie) 5-Cyano-3-[N-(2,3-dihydroxy-propyl)-carbamoyl]-2,4,6-trijod-benzoylchlorid aus als weißes Pulver ; Fp. : 285-288 °C.

3-[N-(2,3-dihydroxypropyl)-carbamoyl]-5-[N-(2,3-dihydroxypropyl)-N-methylcarbamoyl]-2,4,6-trijod-benzonitril

6,6 g 5-Cyano-3-[N-(2,3-dihydroxypropyl)-carbamoyl]-2,4,6-trijod-benzoesäurechlorid werden in 40 ml Dimethylacetamid gelöst und mit 2,3 g N-Methylaminopropandiol (2,3) versetzt. Man läßt eine Stunde bei Raumtemperatur rühren und engt anschließend im Vakuum ein. Das so erhaltene Rohprodukt kann direkt zur Nitrilverseifung eingesetzt werden. Zur Isolierung des reinen Produktes behandelt man die verdünnte wäßrige Lösung des Rohproduktes mit einem Kationen- und einem Anionenaustauscherharz und engt die filtrierte Lösung im Vakuum zur Trockne ein. Man erhält 5,9 g (81 % der Theorie) 3-[N-(2,3-dihydroxypropyl)-carbamoyl]-5-[N-(2,3-dihydroxypropyl)-N-methyl-carbamoyl]-2,4,6-trijod-benzonitril ; Fp. : 215 °C.

3-N,N-Dimethylcarbamoyl-5-[N-(2,3-dihydroxypropyl)-carbamoyl]-2,4,6-trijod-benzoylchlorid

2,78 l Dioxan werden auf 80 °C erhitzt und nacheinander mit 25 g 1-Aminopropandiol-(2,3) und 72,4 g 5-N,N-Dimethylcarbamoyl-2,4,6-trijod-isophthalsäure-dichlorid versetzt. Man hält noch 10 Minuten bei 80 °C, kühlt dann den Ansatz schnell ab, klärt die trübe Lösung durch Filtration über Kieselgur und engt sie im Vakuum zur Trockne ein. Der Rückstand wird mehrfach mit je 300 ml Essigsäureäthylester ausgekocht, abgesaugt und bei 60 °C getrocknet. Man erhält 34,5 g 3-N,N-Dimethylcarbamoyl-5-[N-(2,3-dihydroxypropyl)-carbamoyl]-2,4,6-trijod-benzoylchlorid als weißes Pulver ; Fp. : 145-147 °C (Zersetzung) ; Ausbeute : 45 % der Theorie.

Die nachfolgenden erfindungsgemäßen Beispiele dienen der weiteren Erläuterungen des Erfindungsgegenstandes.

## Beispiel 1

6,3 g 5-Cyano-3-[N-methyl-N-(2-hydroxyäthyl)-carbamoyl]-2,4,6-trijod-benzoesäure werden in einer Lösung aus 15 ml 2 n Natronlauge und 10 ml Wasser 2 Stunden bei 60 °C gerührt. Anschließend wird auf Raumtemperatur abgekühlt, mit 3,25 ml 12 n Salzsäure angesäuert, nach einiger Zeit abgesaugt und mit Wasser nachgespült. Ausbeute : 3,6 g (55,9 % der Theorie) 5-Carbamoyl-3-[N-methyl-N-(2-hydroxyäthyl)-carbamoyl]-2,4,6-trijod-benzoesäure. Fp. : 285/286-288 °C (Zersetzung).

## Beispiel 2

13 g 5-Cyano-3-carbamoyl-2,4,6-trijodbenzoesäure werden in 24,9 ml Wasser und 32,7 ml 2 n NaOH gelöst. Anschließend wird 3 Stunden bei 60 °C gerührt. Nach Abkühlung auf Raumtemperatur wird das Reaktionsgemisch mit 60 ml Wasser verdünnt und mit 7 ml konzentrierter Salzsäure angesäuert. Nach 3 Stunden Rühren bei Raumtemperatur wird der Niederschlag abgesaugt, mit wenig Wasser gewaschen und bei 50 °C im Vakuum getrocknet. Ausbeute : 12,6 (93,9 % der Theorie) 3,5-Bis-carbamoyl-2,4,6-trijod-benzoesäure Fp. : > 300 °C.

## Beispiel 3

Analog Beispiel 2 erhält man aus 3,5-Dicyano-2,4,6-trijod-benzoesäure die 3,5-Bis-Carbamoyl-2,4,6-trijod-benzoesäure. Fp. : > 300 °C.

## Beispiel 4

42,83 g 5-Cyano-3-[N-(2-hydroxyäthyl)-carbamoyl]-2,4,6-trijod-benzoesäure werden in einer Lösung aus 105 ml 2 n Natronlauge und 70 ml Wasser gelöst und 3 Stunden bei 60 °C gerührt. Die filtrierte Lösung wird nach Kühlen in Eis mit 21 ml 12 n Salzsäure angesäuert, der Niederschlag nach ca. 1,5 Stunden abgesaugt, auf der Fritte mit frischem Wasser überschichtet, abgesaugt und im Vakuum bei 50 °C getrocknet. Zur weiteren Reinigung wird das Produkt (40 g) in 160 ml Methanol unter Zusatz von 6,9 ml 11 n Natronlauge gelöst und über Nacht bei Raumtemperatur gerührt. Die Salzfällung wird nach Kühlen in Eis abgesaugt, in 400 ml Wasser gelöst, mit 4 g Kohle gerührt und filtriert. Aus dem Filtrat wird mit 7 ml 12n Salzsäure die reine Säure gefällt. Sie wird nach 2 Stunden abgesaugt, in 200 ml Wasser 30 Minuten ausgerührt, abgesaugt und bei 50 °C getrocknet. Ausbeute : 21,9 g (49,7 % der Theorie) 5-Carbamoyl-3-[N-(2-hydroxyäthyl)-carbamoyl]-2,4,6-trijod-benzoesäure, Fp. : 297/310-312 °C (Zersetzung).

Beispiel 5

77,6 g-Cyano-3-(N-methyl-carbamoyl)-2,4,6-trijod-benzoesäure werden in 145 ml Wasser und 191 ml 2 n Natronlauge gelöst und 3 Stunden bei 60 °C gerührt. Die Lösung wird mit 234 ml Wasser verdünnt, in Eis gekühlt und das Reaktionsprodukt mit 46 ml konzentrierter Salzsäure ausgefällt. Zur Reinigung wird in 400 ml $CH_3OH$ suspendiert und mit 20 ml 7,9 n wäßriger Dimethylaminlösung (ca. 40 %ig) versetzt. Nach vorübergehender Lösung fällt das Salz aus. Nach Rühren und Stehen über 60 Stunden wird das Salz abgesaugt, mit wenig $CH_3OH$ gewaschen und bei 50 °C im Vakuum getrocknet. Das Salz wird in 700 ml Wasser gelöst, 30 Minuten mit 7 g Kohle gerührt, abgesaugt und mit 15 ml konzentrierter Salzsäure ausgefällt. Nach Rühren über Nacht wird der Niederschlag abgesaugt, mit Wasser ausgerührt und bei 50 °C im Vakuum getrocknet. Ausbeute : 61,2 g (76,5 % der Theorie) 5-Carbamoyl-3-N-methyl-carbamoyl-2,4,6-trijod-benzoesäure, Fp. : > 300 °C.

Beispiel 6

59,6 g 5-Cyano-3-N,N-dimethyl-carbamoyl-2,4,6-trijod-benzoesäure werden in 120 ml Wasser und 140 ml 2 n NaOH gelöst und 3 Stunden bei 60 °C gerührt. Die Lösung wird über Aktivkohle filtriert und mit 50 ml konzentrierter Salzsäure unter Rühren versetzt. Nach mehrstündigem Kühlen im Eisbad wird der Niederschlag abgesaugt, mit Wasser gewaschen und bei 50 °C getrocknet. Anschließend wird das Rohprodukt (61 g) in 300 ml Methanol suspendiert und durch Zugabe von 20 ml 7,9 n wäßriger Dimethylaminlösung (ca. 40 %ig) in Lösung gebracht. Nach mehrstündigem Rühren im Eisbad wird das Kristallisat abgesaugt, mit wenig eiskaltem Methanol gewaschen und bei 50 °C im Vakuum getrocknet. Das Salz wird in 600 ml Wasser gelöst, die Lösung über Aktivkohle filtriert und mit 15 ml konzentrierter Salzsäure versetzt. Nach mehrstündigem Rühren im Eisbad wird der Niederschlag abgesaugt, mit Wasser gewaschen und bei 50 °C im Vakuum getrocknet. Ausbeute : 52,2 g (85 % der Theorie) 5-Carbamoyl-3-N, N-dimethyl-carbamoyl-2,4,6-trijod-benzoesäure, Fp. : 255 °C (Zersetzung).

Beispiel 7

8,94 g 5-Cyano-3-acetylaminomethyl-2,4,6-trijod-benzoesäure werden in einer Lösung aus 22,5 ml 2 n Natronlauge und 15 ml Wasser 3 Stunden bei 60 °C gerührt. Anschließend wird in Eis Abgekühlt, mit 4,5 ml 12 n Salzsäure angesäuert, der Niederschlag nach 1,5 Stunden abgesaugt, nachgewaschen, abgesaugt und im Vakuum bei 50 °C getrocknet. Ausbeute : 8,7 g (94,5 % der Theorie) 5-Carbamoyl-3-acetylaminomethyl-2,4,6-trijod-benzoesäure, Fp. : 228-230 °C (Zersetzung).

Beispiel 8

27,8 g 5-Cyano-3-hydroxymethyl-2,4,6-trijod-benzoesäure werden in 120 ml Wasser suspendiert und durch Zugabe von 10 g Ätznatron in Lösung gebracht. Man hält den Ansatz 3 Stunden auf 60 °C und fällt anschließend in 50 ml halbkonzentrierte Schwefelsäure. Nach mehrstündigem Rühren im Eisbad wird der Niederschlag abgesaugt, mit Wasser gewaschen und bei 50 °C getrocknet. Man erhält 25 g 5-Carbamoyl-3-hydroxymethyl-2,4,6-trijod-benzoesäure als weißes Pulver mit einem oberhalb 280 °C liegenden Zersetzungspunkt ; Ausbeute : 88 % der Theorie.

Beispiel 9

12,3 g 3,5-Bis-(N-methylcarbamoyl)-2,4,6-trijod-benzoesäureamid werden in 80 ml Eisessig suspendiert. Bei Raumtemperatur wird unter Rühren eine Mischung von 1,66 g Natriumnitrit in 12 ml konzentrierte Schwefelsäure (unter Eiskühlung hergestellt) in kleinen Portionen hinzugefügt, wobei die Temperatur von 24 °C auf 42 °C steigt. Anschließend wird 3 Stunden bei Raumtemperatur, 30 Minuten bei 50 °C und 90 Minuten bei 70 °C gerührt. Nach Rühren über Nacht bei Raumtemperatur wird das Reaktionsgemisch in 400 ml Wasser eingerührt. Der Niederschlag wird nach 4 Stunden abgesaugt, mit Wasser gewaschen und bei 50 °C im Vakuum getrocknet. Das Rohprodukt (11,9 g) wird in 110 ml Wasser und 12 ml 2 n Natronlauge gelöst, mit 2 n Essigsäure auf pH 6,5 eingestellt und 30 Minuten mit 1 g Kohle gerührt. Nach Absaugen der Kohle wird die Lösung mit 16 ml 2 n Salzsäure ausgefällt. Nach Absaugen und Trocknen bei 50 °C im Vakuum werden 10,7 g (87 % der Theorie) 3,5-Bis-(N-methylcarbamoyl)-2,4,6-trijod-benzoesäure vom Fp. > 300 °C erhalten.

Beispiel 10

112 g 3,5-Bis-[N-(2-hydroxyäthyl)-carbamoyl]-2,4,6-trijod-benzoesäureamid werden in 665 ml Eisessig mit einer Mischung von 13,8 g Natriumnitrit in 100 ml konzentrierter Schwefelsäure innerhalb 25 Minuten versetzt. Anschließend wird 2 Stunden bei Raumtemperatur 30 Minuten bei ca. 50 °C und 1 Stunde bei ca. 70 °C gerührt. Nach Abkühlung auf Raumtemperatur wird die Lösung in 1,66 l Wasser eingerührt. Nach Rühren über Nacht bei Raumtemperatur wird der Niederschlag abgesaugt, mit wenig

Wasser gewaschen und bei 50 °C im Vakuum getrocknet. Ausbeute : 75,4 g partiell acetylierte Verbindung. Diese Verbindung wird in 225 ml Wasser suspendiert und unter Zugabe von insgesamt 30 ml konzentrierter NaOH bei pH 10-11 innerhalb 30 Minuten auf dem Dampfbad verseift. Die warme Lösung wird mit 35 ml konzentrierter Salzsäure angesäuert. Die Fällung wird nach Rühren über Nacht abgesaugt, mit Wasser gewaschen und bei 50 °C im Vakuum getrocknet. Ausbeute : 62,5 g (55,9 % der Theorie) 3,5-Bis-[N-(2-hydroxyäthyl)-carbamoyl]-2,4,6-trijod-benzoesäure, Fp. : 288-290 °C.

Beispiel 11

Eine Suspension von 33,6 g 3,5-Bis-[N-(2-hydroxyäthyl)-carbamoyl]-2,4,6-trijod-benzamid in 60 ml Dimethylformamid wird bei Raumtemperatur unter Rühren innerhalb 5 Minuten mit 73 ml einer Nitrosylchloridlösung in Dimethylformamid (7,9 g Nitrosylchlorid) versetzt, wobei eine schwache Wärmetönung auftritt. Nach 2 tägigem Rühren bei Raumtemperatur wird die gelbliche Suspension mit 350 ml Wasser versetzt und 30 Minuten auf dem Dampfbad gerührt. Die Lösung wird an der Wasserdampfstrahlpumpe bei 60 °C weitgehend eingeengt, der Rückstand mit 200 ml Wasser verdünnt, mit 7,5 ml konzentrierter Salzsäure versetzt und 30 Minuten auf dem Dampfbad gerührt. Dabei fällt unter schwacher Gasentwicklung ein Niederschlag aus. Nach Rühren über Nacht bei Raumtemperatur wird der Niederschlag abgesaugt, mit wenig Wasser gewaschen und bei 50 °C im Vakuum getrocknet. Ausbeute : 24,5 g (72,7 % der Theorie) 3,5-Bis-[N-(2-hydroxyäthyl)-carbamoyl]-2,4,6-trijod-benzoesäure, Fp. : 289-291 °C.

Beispiel 12

Analog Beispiel 11 werden 32 g 3-[N-(2-Hydroxyäthyl)-carbamoyl]-5-N-methylcarbamoyl-2,4,6-trijodbenzoesäureamid in 60 ml Dimethylformamid mit 7,9 g Nitrosylchlorid umgesetzt. Ausbeute : 26,5 g (82 % der Theorie) 3-[N-(2-Hydroxyäthyl)-carbamoyl]-5-N-methylcarbamoyl-2,4,6-trijod-benzoesäure ; Fp. : 271-274 °C.

Beispiel 13

7 g 3-N,N-Dimethylcarbamoyl-5-[N-(2,3-dihydroxypropyl)-carbamoyl]-2,4,6-trijod-benzoylchlorid werden in 15 ml Dimethylsulfoxid gelöst. Nach zugabe von 5 ml 2 n-Natronlauge läßt man den Ansatz über Nacht bei Raumtemperatur stehen und engt dann im Vakuum zur Trockne ein. Der Rückstand wird in 20 ml Wasser aufgenommen, über Aktivkohle filtriert und mit überschüssiger konzentrierter Salzsäure unter Rühren versetzt. Nach mehrstündigem Rühren wird der gebildete Niederschlag abgesaugt, mit wenig eiskaltem Wasser gewaschen und bei 50 °C getrocknet. Man erhält 6,2 g (90 % der Theorie) 3-N,N-Dimethylcarbamoyl-5-N-(2,3-dihydroxypropyl)-carbamoyl-2,4,6-trijod-benzoesäure als weißes Pulver vom Schmelzpunkt 255-258 °C (Zersetzung).

Beispiel 14

6 g 5-Cyano-3-N,N-dimethylcarbamoyl-2,4,6-trijod-benzoesäure werden in 30 ml Essigsäureanhydrid suspendiert. Nach Zugabe von 0,5 ml 80 %iger Perchlorsäure hält man den Ansatz 3 Stunden bei 90-95 °C, filtriert über Aktivkohle, tropft unter guter Kühlung in 200 ml Wasser ein und bringt durch Zugabe von Natriumcarbonat bei pH 6 in Lösung. Nach Behandeln mit Aktivkohle wird die Lösung mit überschüssiger konzentrierter Salzsäure versetzt und der Niederschlag nach Absaugen und Waschen mit Wasser in 40 ml Aceton gelöst. Nach mehrstündigem Rühren im Eisbad wird das Kristallisat abgesaugt, mit wenig eiskaltem Aceton gewaschen und bei 50 °C getrocknet. Man erhält 4,5 g (68 % der Theorie) 5-Acetylaminocarbonyl-3-N,N-dimethylcarbamoyl-2,4,6-trijod-benzoesäure als weißes Pulver mit einem oberhalb 280 °C liegenden Zersetzungpunkt.

Beispiel 15

Analog Beispiel 11 werden 15 g 5-Carbamoyl-3-N-methyl-carbamoyl-2,4,6-trijod-benzoesäure-(2,3-dihydroxy-propyl)-ester in 30 ml Dimethylformamid mit 4 g Nitrosylchlorid umgesetzt und in gleicher Weise aufgearbeitet. Man erhält 11 g (70 % der Theorie) 5-N-Methylcarbamoyl-2,4,6-trijod-isophthalsäure-mono-(2,3-dihydroxy-propyl)-ester ; Fp. : 232-240 °C.

Beispiel 16

| | |
|---|---|
| 5-Carbamoyl-2,4,6-trijod-isophthalsäure-monomethylamid | 472,71 g |
| N-Methyl-glucamin | 153,83 g |
| Calciumdinatriumsalz der Äthylendiaminotetraessigsäure | 0,10 g |
| bidestilliertes Wasser bis zum Volumen von | 1 000 ml |

Ausführung : Die Komponenten werden vereinigt, mit bidestilliertem Wasser auf 1 000 ml aufgefüllt

und anschließend hitzesterilisiert. Jodgehalt : 300 mg/ml.

Beispiel 17

198 g 5-Cyano-2,4,6-trijod-isophthalsäure-mono-(2-hydroxy-1-hydroxymethyl-äthyl)amid werden in 1 500 ml Wasser suspendiert und mit 30 g Ätznatron versetzt. Die Lösung wird 3 Stunden bei 60 °C gerührt, 30 Minuten mit Aktivkohle behandelt, filtriert und unter Kühlung im Eisbad durch Zugabe von konzentrierter Salzsäure auf pH 0,1 gebracht. Nach Rühren über Nacht unter Kühlung saugt man das ausgeschiedene Kristallisat ab, wäscht es mit wenig eiskaltem Wasser und trocknet es bei 50 °C. Man erhält 167 g (82 % der Theorie) 5-Carbamoyl-3-[N-(2-hydroxy-1-hydroxymethyläthyl)-carbamoyl]-2,4,6-trijod-benzoesäure als weißes Pulver mit einem oberhalb 280 °C liegenden Zersetzungspunkt.

Beispiel 18

46,5 g 5-Cyano-2,4,6-trijod-isophthalsäure-mono-(2-methoxyäthyl)amid werden in 180 ml Wasser suspendiert und durch Zugabe von 9 g Ätznatron in Lösung gebracht. Dann erwärmt man die Lösung 3 Stunden auf 60 °C, behandelt sie 30 Minuten mit Aktivkohle und bringt nach Filtration durch Zugabe von konzentrierter Salzsäure auf pH 0,1. Nach mehrstündigem Rühren im Eisbad wird der ausgeschiedene Niederschlag abgesaugt, mit Wasser gewaschen und bei 50 °C getrocknet. Man erhält 43 g (90 % der Theorie) 5-Carbamoyl-3-[N-(2-methoxyäthyl)-carbamoyl]-2,4,6-trijod-benzoesäure als weißes Pulver vom Fp. : 286 °C (Zersetzung).

**Ansprüche**

1. Verbindungen der allgemeinen Formel I

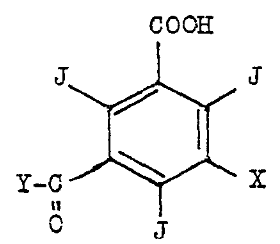

(I)

worin

X den Rest $—CONR_1R_2$, $—CH_2NHAcyl$ oder $—CH_2OH$,

Y den Rest $—NR_3R_4$, —NH · Acyl oder $OR_5$ darstellen,

$R_1$, $R_2$, $R_3$ und $R_4$ gleich oder verschieden sind und ein Wasserstoffatom oder einen gerad- oder verzweigtkettigen Alkylrest mit 1 bis 6 Kohlenstoffatomen oder einen gerad- oder verzweigtkettigen Mono- oder Polyhydroxyalkylrest mit 2 bis 8 Kohlenstoffatomen und 1 bis 5 Hydroxylgruppen bedeuten,

$R_5$ dieselbe Bedeutung wie $R_1$ bis $R_4$ hat, ausgenommen die Bedeutung als Wasserstoffatom,

Acyl den Rest einer aliphatischen Carbonsäure mit 2 bis 6 Kohlenstoffatomen, deren Alkylrest mit 1 bis 5 Hydroxylgruppen substituiert sein kann, darstellt, wobei in den Alkylresten $R_1$ bis $R_5$ und im Alkylrest anwesende Hydroxylgruppen auch funktionell abgewandelt sein können, und deren physiologisch verträgliche Salze mit anorganischen oder organischen Basen.

2. 5-Carbamoyl-3-[N-methyl-N-(2-hydroxyäthyl)-carbamoyl]-2,4,6-trijod-benzoesäure.
3. 5-Carbamoyl-3-[N-(2-hydroxyäthyl)-carbamoyl]-2,4,6-trijod-benzoesäure.
4. 5-Carbamoyl-3-N-methylcarbamoyl-2,4,6-trijod-benzoesäure.
5. 5-Carbamoyl-3-N,N-dimethylcarbamoyl-2,4,6-trijod-benzoesäure.
6. 3,5-Bis-[N-(2-hydroxyäthyl)-carbamoyl]-2,4,6-trijod-benzoesäure.
7. 5-N-Methylcarbamoyl-3-[N-(2-hydroxyäthyl)-carbamoyl]-2,4,6-trijod-benzoesäure.
8. 5-Carbamoyl-3-[N-(2-methoxyäthyl)-carbamoyl]-2,4,6-trijod-benzoesäure.
9. Röntgenkontrastmittel, enthaltend eine schattengebende Substanz gemäß der Ansprüche 1-8.
10. Verfahren zur Herstellung von Verbindungen der allgemeinen Formel I

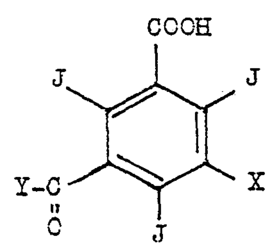

(I)

worin

X den Rest $—CONR_1R_2$, $—CH_2NHAcyl$ oder $—CH_2OH$,

Y den Rest $—NR_3R_4$, $—NH \cdot Acyl$ oder $OR_5$ darstellen,

$R_1$, $R_2$, $R_3$ und $R_4$ gleich oder verschieden sind und ein Wasserstoffatom oder einen gerad- oder verzweigtkettigen Alkylrest mit 1 bis 6 Kohlenstoffatomen oder einen gerad- oder verzweigtkettigen Mono- oder Polyhydroxyalkylrest mit 2 bis 8 Kohlenstoffatomen und 1 bis 5 Hydroxylgruppen bedeuten,

$R_5$ dieselbe Bedeutung wie $R_1$ bis $R_4$ hat, ausgenommen die Bedeutung als Wasserstoffatom,

Acyl den Rest einer aliphatischen Carbonsäure mit 2 bis 6 Kohlenstoffatomen, deren Alkylrest mit 1 bis 5 Hydroxylgruppen substituiert sein kann, darstellt, wobei in den Alkylresten $R_1$ bis $R_5$ und im Alkylrest anwesende Hydroxylgruppen auch funktionell abgewandelt sein können, und deren physiologisch verträgliche Salze mit anorganischen oder organischen Basen, dadurch gekennzeichnet, daß man in an sich bekannter Weise

a) eine Verbindung der allgemeinen Formel A

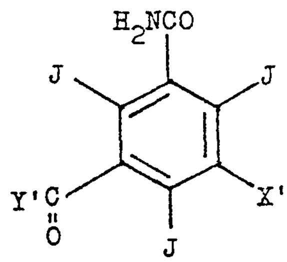

(A)

worin

X' die Gruppen $—CONR_1'R_2'$, $—CH_2OH$ oder $—CH_2NHAcyl$,

Y' die Gruppen $—NR_3'R_4'$, $—NHAcyl$ oder $—OR_5$ bedeuten und

$R_1'$, $R_2'$, $R_3'$, $R_4'$, $R_5$ und Acyl die obenangegebenen Bedeutungen für $R_1$, $R_2$, $R_3$, $R_4$ und $R_5$ besitzen, jedoch sowohl $R_1'$ und $R_2'$ als auch $R_3'$ und $R_4'$ nicht gleichzeitig Wasserstoff darstellen und Acyl die obengenannte Bedeutung besitzt, mit einem Diazotierungsreagenz umsetzt oder

b) in einer Verbindung der allgemeinen Formel B

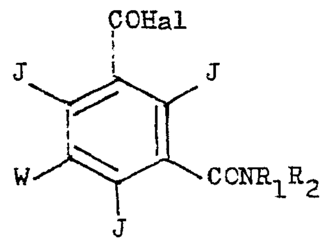

(B)

worin

W die Gruppen $—CONR_3R_4$, $—CONHAcyl$, $—CH_2OH$ oder $—CH_2NHAcyl$,

Hal ein Brom- oder Chloratom bedeuten und

$R_1$, $R_2$, $R_3$, $R_4$ und Acyl die obengenannten Bedeutungen besitzen, die Carbonsäurehalogenidgruppe hydrolysiert oder

c) eine Verbindung der allgemeinen Formel C

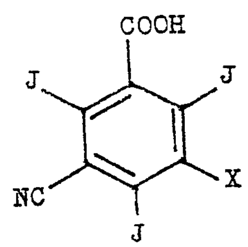

(C)

worin

X die obengenannte Bedeutung besitzt, mit einem reaktionsfähigen Derivat einer aliphatischen Carbonsäure AcylOH umsetzt, worin Acyl die obengenannte Bedeutung besitzt, oder der partiellen Hydrolyse unterwirft und gegebenenfalls die so erhaltene 5-Carbamoylverbindung der Formel I, worin $R_1$ und $R_2$ nicht gleichzeitig Wasserstoff sind, diazotiert, anschließend mit einer Base der Formel $HNR_3R_4$ (mit $R_3$ und $R_4$ in der obengenannten Bedeutung) partiell amidiert und das so erhaltene Monocarbonsäurechlorid hydrolysiert und gewünschtenfalls anschließend die erhaltenen Verbindungen der Formel I N-alkyliert und/oder Schutzgruppen abspaltet und/oder durch Umsetzung mit anorganischen oder organischen Basen Salze herstellt.

11. Verfahren nach Anspruch 10, dadurch gekennzeichnet, daß man die Verbindungen der Ansprüche 2-8 herstellt.

**Claims**

1. Compounds of the general formula I

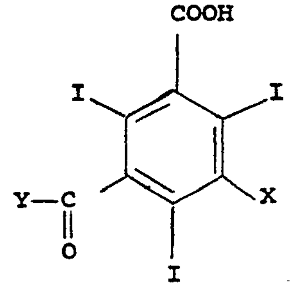

(I)

in which

X represents the radical —$CONR_1R_2$, —$CH_2NHacyl$ or —$CH_2OH$,

Y represents the radical —$NR_3R_4$, —NHacyl or $OR_5$,

$R_1$, $R_2$, $R_3$ and $R_4$ are the same or different and represent a hydrogen atom or a straight-chain or branched-chain alkyl radical having from 1 to 6 carbon atoms or a straight-chain or branched-chain mono- or polyhydroxyalkyl radical having from 2 to 8 carbon atoms and from 1 to 5 hydroxy groups,

$R_5$ has the same meaning as $R_1$ to $R_4$, with the exception of the meaning of a hydrogen atom,

acyl represents the radical of an aliphatic carboxylic acid having from 2 to 6 carbon atoms, the alkyl radical of which may be substituted by from 1 to 5 hydroxy groups, it being possible for hydroxy groups present in the alkyl radicals $R_1$ to $R_5$ and in the acyl radical also to be functionally modified, and their physiologically tolerable salts with inorganic or organic bases.

2. 5-Carbamoyl-3-[N-methyl-N-(2-hydroxyethyl)-carbamoyl]-2,4,6-triiodobenzoic acid.

3. 5-Carbamoyl-3-[N-(2-hydroxyethyl)-carbamoyl]-2,4,6-triiodobenzoic acid.

4. 5-Carbamoyl-3-N-methylcarbamoyl-2,4,6-triiodobenzoic acid.

5. 5-Carbamoyl-3-N,N-dimethylcarbamoyl-2,4,6-triiodobenzoic acid.

6. 3,5-bis[N-(2-hydroxyethyl)-carbamoyl]-2,4,6-triiodobenzoic acid.

7. 5-N-Methylcarbamoyl-3-[N-(2-hydroxyethyl)-carbamoyl]-2,4,6-triiodobenzoic acid.

8. 5-Carbamoyl-3-[N-(2-methoxyethyl)-carbamoyl]-2,4,6-triiodobenzoic acid.

9. X-ray contrast agents containing a shadow-forming substance according to claims 1 to 8.

10. Process for the preparation of compounds of the general formula I

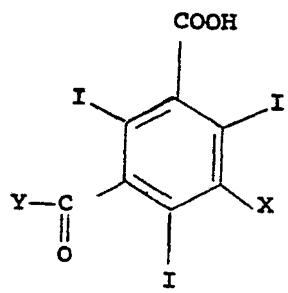

(I)

in which

X represents the radical —$CONR_1R_2$, —$CH_2NHacyl$ or —$CH_2OH$,

Y represents the radical —$NR_3R_4$, —NHacyl or $OR_5$,

$R_1$, $R_2$, $R_3$ and $R_4$ are the same or different and repressent a hydrogen atom or a straight-chain or branched-chain alkyl radical having from 1 to 6 carbon atoms or a straight-chain or branched-chain mono- or polyhydroxyalkyl radical having from 2 to 8 carbon atoms and from 1 to 5 hydroxy groups,

$R_5$ has the same meaning as $R_1$ to $R_4$, with the exception of the meaning of a hydrogen atom,

acyl represents the radical of an aliphatic carboxylic acid having from 2 to 6 carbon atoms, the alkyl radical of which may be substituted by from 1 to 5 hydroxy groups, it being possible for hydroxy groups present in the alkyl radicals $R_1$ to $R_5$ and in the acyl radical also to be functionally modified, and their physiologically tolerable salts with inorganic or organic bases, characterised in that in a manner known *per se*

a) a compound of the general formula A

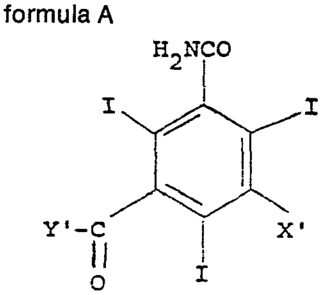

(A)

in which

X' represents the groups —$CONR_1'R_2'$, —$CH_2OH$ or —$CH_2NH$-acyl,

Y' represents the groups —$NR_3'R_4'$, —NHacyl or —$OR_5$ and

$R_1'$, $R_2'$, $R_3'$, $R_4'$, $R_5$ and acyl have the meanings given above for $R_1$, $R_2$, $R_3$, $R_4$ and $R_5$, but neither $R_1'$ and $R_2'$ nor $R_3'$ and $R_4'$ simultaneously represent hydrogen, and acyl has the above-mentioned meaning, is reacted with a diazotising reagent or

b) in a compound of the general formula B

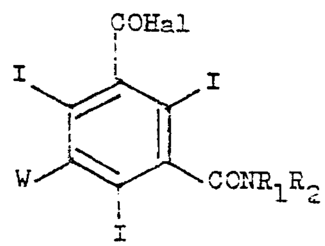

(B)

in which

W represents the groups —$CONR_3R_4$, —CONHacyl, —$CH_2OH$ or —$CH_2NHacyl$,

Hal represents a bromine or chlorine atom and

$R_1$, $R_2$, $R_3$, $R_4$ and acyl have the above-mentioned meanings, the carboxylic acid halide group is hydrolysed or

c) a compound of the general formula C

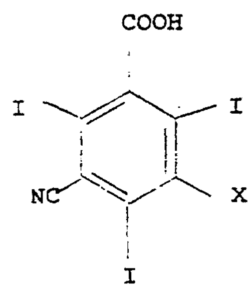

(C)

in which

X has the above-mentioned meaning, is reacted with a reactive of an aliphatic carboxylic acid acylOH, in which acyl has the above-mentioned meaning, or is subjected to partial hydrolysis and the resulting 5-carbamoyl compound of the formula I, in which $R_1$ and $R_2$ are not simultaneously hydrogen, is optionally diazotised, then partially amidated with a base of the formula $HNR_3R_4$ ($R_3$ and $R_4$ having the above-mentioned meaning) and the resulting monocarboxylic acid chloride is hydrolysed and, if desired, the resulting compounds of the formula I are then N-alkylated and/or protective groups are split off and/or salts are prepared by reaction with inorganic or organic bases.

11. Process according to claim 10, characterised in that the compounds of claims 2 to 8 are prepared.

**Revendications**

1. Composés répondant à la formule générale (I)

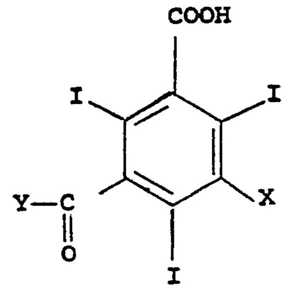

(I)

dans laquelle

X représente un radical $—CONR_1R_2$, $—CH_2NH \cdot Acyl$ ou $—CH_2OH$,

Y représente un radical $—NR_3R_4$, $—NH \cdot Acyl$ ou $—OR_5$,

$R_1$, $R_2$, $R_3$ et $R_4$ peuvent être identiques ou différents et représentent chacun un atome d'hydrogène, un radical alkyle, linéaire ou ramifié, contenant de 1 à 6 atomes de carbone, ou un radical mono- ou polyhydroxyalkyle, linéaire ou ramifié, contenant de 2 à 8 atomes de carbone et portant de 1 à 5 groupes hydroxy,

$R_5$ a la même signification que $R_1$ à $R_4$ mais ne peut représenter un atome d'hydrogène,

Acyl représente le radical d'un acide carboxylique aliphatique contenant de 2 à 6 atomes de carbone et dont le radical alkyle peut porter de 1 à 5 groupes hydroxy, les groupes hydroxy présents dans les radicaux alkyles $R_1$ à $R_5$ et dans le radical alkyle pouvant également être sous la forme de dérivés fonctionnels, ainsi que les sels acceptables du point de vue physiologique qu'ils forment avec des bases minérales ou organiques.

2. Acide carbamoyl-5 [N-méthyl-N-(hydroxy-2 éthyl)-carbamoyl]-3 triiodo-2,4,6 benzoïque.

3. Acide carbamoyl-5 [N-(hydroxy-2 éthyl)-carbamoyl]-3 triiodo-2,4,6 benzoïque.

4. Acide carbamoyl-5 N-méthylcarbamoyl-3 triiodo-2,4,6 benzoïque.

5. Acide carbamoyl-5 N,N-diméthylcarbamoyl-3 triiodo-2,4,6 benzoïque.

6. Acide bis-[N-(hydroxy-2 éthyl)-carbamoyl]-3,5 triiodo-2,4,6 benzoïque.

7. Acide N-méthylcarbamoyl-5 [N-(hydroxy-2 éthyl)-carbamoyl]-3 triiodo-2,4,6 benzoïque.

8. Acide carbamoyl-5 [N-(méthoxy-2 éthyl)-carbamoyl]-3 triiodo-2,4,6 benzoïque.

9. Agent de contraste pour rayons X qui contient un composé opacifiant selon l'une quelconque des revendications 1 à 8.

10. Procédé de préparation de composés répondant à la formule générale (I)

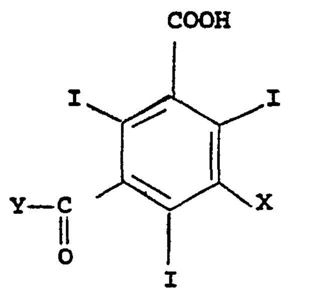

(I)

dans laquelle

X représente un radical $—CONR_1R_2$, $—CH_2NH \cdot Acyl$ ou $—CH_2OH$,

Y représente un radical $—NR_3R_4$, $—NH \cdot Acyl$ ou $—OR_5$,

$R_1$, $R_2$, $R_3$ et $R_4$ peuvent être identiques ou différents et représentent chacun un atome d'hydrogène, un radical alkyle, linéaire ou ramifié, contenant de 1 à 6 atomes de carbone, ou un radical mono- ou polyhydroxyalkyle, linéaire ou ramifié, contenant de 2 à 8 atomes de carbone et portant de 1 à 5 groupes hydroxy,

$R_5$ a la même signification que $R_1$ à $R_4$ mais ne peut représenter un atome d'hydrogène,

Acyl représente le radical d'un acide carboxylique aliphatique contenant de 2 à 6 atomes de carbone et dont le radical alkyle peut porter de 1 à 5 groupes hydroxy, les groupes hydroxy présents dans les radicaux alkyles $R_1$ à $R_5$ et dans le radical alkyle pouvant également être sous la forme de dérivés fonctionnels, ainsi que des sels acceptables du point de vue physiologique qu'ils forment avec des bases minérales ou organiques, procédé caractérisé en ce que, en opérant de manière connue

a) on fait réagir un composé répondant à la formule générale (A)

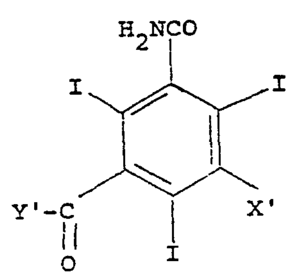

(A)

dans laquelle

X' représente un radical $—CONR_1'R_2'$, $—CH_2OH$ ou $—CH_2NHAcyl$, et

Y' représente un radical $—NR_3'R_4'$, $—NHAcyl$ ou $—OR_5$, et

$R_1'$, $R_2'$, $R_3'$, $R_4'$, $R_5$ et Acyl ont les significations précédemment données pour $R_1$, $R_2$, $R_3$, $R_4$ et $R_5$ mais $R_1'$ et $R_2'$, de même que $R_3'$ et $R_4'$, ne pouvant pas représenter chacun en même temps l'hydrogène, et Acyl a la signification indiquée plus haut, avec un agent diazotant, ou

b) dans un composé répondant à la formule générale (B)

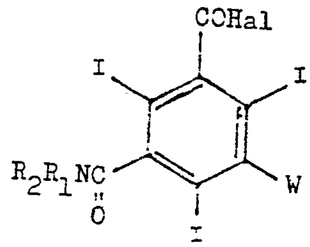

(B)

dans laquelle

W représente un radical $—CONR_3R_4$, —CONHAcyl, $—CH_2OH$ ou $—CH_2NHAcyl$,

Hal représente un atome de brome ou de chlore et

$R_1$, $R_2$, $R_3$, $R_4$ et Acyl ont les significations précédemment données, on hydrolyse le radical halogénocarbonyle, ou

c) on fait réagir un composé répondant à la formule générale (C)

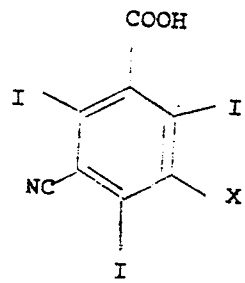

(C)

dans laquelle

X a la signification précédemment donnée, avec un dérivé réactif d'un acide carboxylique aliphatique AcylOH dans lequel Acyl a la signification indiquée ci-dessus, ou on le soumet à une hydrolyse partielle et éventuellement on diazote le composé à radical carbamoyle en 5 ainsi obtenu, qui répond à la formule (I) dans laquelle $R_1$ et $R_2$ ne représentent pas chacun en même temps l'hydrogène, puis on amide partiellement avec une base de formule $NHR_3R_4$ (dans laquelle $R_3$ et $R_4$ ont les significations précédemment données) et on hydrolyse le composé mono-(chlorocarbonylique) ainsi obtenu, après quoi, si on le juge bon, on alkyle à l'azote les composés de formule (I) obtenus et/ou on élimine d'éventuels radicaux protecteurs et/ou on prépare des sels par réaction avec des bases minérales ou organiques.

11. Procédé selon la revendication 10, caractérisé en ce qu'on prépare les composés des revendications 2 à 8.